# EUROPÄISCHE PATENTSCHRIFT

(11) **EP 1 001 701 B1**
(45) Veröffentlichungstag und Bekanntmachung des Hinweises auf die Patenterteilung: **06.10.2004**
(21) Anmeldenummer: 98948737.6
(22) Anmeldetag: 06.08.1998
(51) Int. Cl.: A61B 5/00

(54) **ANALYSEVORRICHTUNG ZUR IN-VIVO-ANALYSE IM KÖRPER EINES PATIENTEN**
ANALYTICAL DEVICE FOR IN VIVO ANALYSIS IN THE BODY OF A PATIENT
DISPOSITIF D'ANALYSE POUR L'ANALYSE IN VIVO DANS LE CORPS D'UN PATIENT

(30) Priorität: 09.08.1997 DE 19734618; 09.08.1997 DE 19734617
(43) Veröffentlichungstag der Anmeldung: 24.05.2000
(73) Patentinhaber: Roche Diagnostics GmbH, 68298 Mannheim (DE); Fraunhofer-Gesellschaft zur Förderung der angewandten Forschung e.V., 80686 München (DE)
(72) Erfinder: HAAR, Hans-Peter, D-69168 Wiesloch (DE); WERNER, Gerhard, D-69469 Weinheim (DE); BOECKER, Dirk, D-69120 Heidelberg (DE); LAMBRECHT, Armin, D-79232 March (DE); KASTNER, Joachim, D-44139 Dortmund (DE)
(74) Vertreter: Pfeifer, Hans-Peter, Dr.
(86) Internationale Anmeldenummer: PCT/DE1998/002262
(87) Internationale Veröffentlichungsnummer: WO 1999/007277

(56) Entgegenhaltungen:
- EP-A- 0 404 562
- WO-A-93/07469
- WO-A-95/10223
- WO-A-96/10198
- US-A- 5 452 716

## Beschreibung

Die Erfindung betrifft eine Analysevorrichtung zur Bestimmung eines Analyten im Körper eines (menschlichen, u.U. auch tierischen) Patienten mit einer Meßsonde, die eine in die Haut einstechbare Kanüle aufweist.

Die Konzentration der Komponenten von Körperflüssigkeiten (Analyten) wird für medizinische Zwecke praktisch ausschließlich mittels Reagenzien bestimmt. Dabei wird eine Probe der Körperflüssigkeit (insbesondere Blut) entnommen und im Labor in vitro analysiert. Obwohl diese Verfahren ständig verbessert wurden und mittlerweile für wichtige Analyten, wie insbesondere die Blutglucose, kleine handliche Analysesysteme zur Verfügung stehen, ist nachteilig, daß für jede einzelne Untersuchung eine Blutentnahme erforderlich und keine kontinuierliche Messung möglich ist.

Zur kontinuierlichen Messung durch Eintauchen in eine Probenflüssigkeit sind faseroptische chemische Sensoren (fibre optic chemical sensors; FOCS) bekannt, die darauf basieren, daß durch eine Lichtleitfaser die Absorption oder Lumineszenz eines Indikatormoleküls beobachtet wird, das an der Spitze der Lichtleitfaser oder in einer diese umgebenden Umhüllung lokalisiert ist. Unter anderem wurden FOCS auch zur kontinuierlichen Messung von Analyten im Blut des Patienten, beispielsweise mittels eines in die Ader eingeführten Katheders, vorgeschlagen. Derartige Vorrichtungen sind in folgenden Druckschriften beschrieben:
a) US-Patent 5,127,077
b) EP 0 589 862 A2
c) US-Patent 4,846,548

Es gibt deshalb bereits seit langem Bemühungen, reagenzienfreie Analyseverfahren zu entwickeln, die überwiegend auf den Prinzipien der Spektroskopie basieren. Konventionelle Absorptionsspektroskopie mittels einer Transmissionsmessung ist an Blut allerdings in weiten Teilen des Spektrums nicht möglich, weil es sehr stark absorbierende Substanzen enthält (insbesondere Hämoglobin), die die charakteristischen Spektralbanden der gesuchten Analyten überlagern. Selbst wenn man das Hämoglobin durch Zentrifugation entfernt, bleibt eine sehr starke störende optische Absorption in den besonders interessanten Bereichen des Infrarot-Spektrums.

Zur Untersuchung wässriger biologischer Flüssigkeiten, insbesondere Blut, wurden deshalb die Möglichkeiten der ATR(Attenuated Total Reflection)-Spektroskopie untersucht. Verwiesen sei insbesondere auf folgende Publikationen:
1) Y. Mendelson: "Blood Glucose Measurement by Multiple Attenuated Total Reflection and Infrared Absorption Spectroscopy", IEEE Transactions on Biomedical Engineering, 1990, 458-465
2) H.M. Heise et al.: "Multicomponent Assay for Blood Substrates in Humas Plasma by Mid-Infrared Spectroscopy and its Evaluation for Clinical Analysis", Applied Spectroscopy, 1994, 85-95
3) R. Simhi et al.: "Multicomponent Analysis of Human Blood using Fiberoptic Evanescent Wave Spectroscopy", SPIE Proc. Vol. 2331: Medical Sensors II and Fiber Optic Sensors, 09/06-09/10/94, Lille, France, A.V. Scheggi et al. (Eds.), ISBN 0-8194-1664-9, published 1995, pp. 166-172

Diese Literaturstellen zeigen, daß es mit der ATR-Spektroskopie grundsätzlich möglich ist, wichtige Analyten im Blut, insbesondere Glucose, reagenzienfrei spektroskopisch zu bestimmen. Die ATR-Spektroskopie basiert darauf, daß Licht in einem Lichtleiter transportiert wird, dessen äußere Oberfläche in Kontakt mit der Probe steht. Der Brechungsindex im Lichtleiter (in Relation zu dem Brechungsindex in der Probe) und der Reflexionswinkel des Lichtes an der Grenzfläche müssen dabei so gewählt sein, daß Totalreflexion des Lichtes stattfindet. Bei der Totalreflexion dringt eine evaneszente Welle in das benachbarte Medium (die Probe) ein. Eine dabei auftretende Absorption führt zu einer Schwächung der Intensität des in dem Lichtleiter transportierten Lichtes. Diese Lichtschwächung kann wellenlängenabhängig ausgewertet werden, um aus dem Spektrum Informationen über die Gegenwart des Analyten in der Probe zu gewinnen. Nähere Einzelheiten können der einschlägigen Literatur, insbesondere den obigen Zitaten 1) bis 3) entnommen werden.

Routinemäßig werden für ATR-Messungen spezielle ATR-Meßzellen eingesetzt, bei denen der Lichtleiter eine prismatische Form hat. Als Alternative wurden vielfach bereits faserförmige Lichtleiter vorgeschlagen. Ein Beispiel, das sich auf die medizinische Analytik von Blutbestandteilen bezieht, ist das Zitat 3).

In der Literaturstelle

### 4) US-Patentschrift 5,436,454

ist eine Vorrichtung beschrieben, mit der die ATR-Spektroskopie in vivo im Blut eines Patienten möglich sein soll. Zu diesem Zweck wird eine dünne Kanüle ähnlich einer Spritzennadel verwendet, die für in vivo-Messungen durch die Haut des Patienten in ein Blutgefäß eingeführt werden kann. In der Kanüle verläuft eine dünne Lichtleitfaser bis an deren Spitze, wird dort in einer engen Schleife in die Gegenrichtung zurückgebogen und läuft in der Kanüle zurück. Durch einen in der Kanüle verlaufenden Lichtleiterschenkel wird Meßlicht zu der Schleife transportiert. Durch den zweiten Schenkel wird es zu einem Detektor zurückgeleitet. Die Kanüle hat einen Durchmesser von etwa 3 mm und eine Innenbohrung von etwa 2 mm zur Aufnahme von Lichtleitfasern mit etwa 0,7 mm bis 1 mm Durchmesser. In der Druckschrift wird erläutert, daß in dem Bereich der Schleife sehr viel mehr Reflexionen des in dem Lichtleiter transportierten Lichts stattfinden, als in dessen geraden Abschnitten. Dadurch wird im Bereich der Schleife eine wesentlich erhöhte Empfindlichkeit erreicht. An der Spitze der Kanüle, aus der die Schleife im Meßzustand geringfügig herausragt, wird durch eine Abdichtung das Eindringen der Probe in die Kanüle verhindert. Dadurch wird die Messung ausschließlich auf den Bereich der Schleife konzentriert. Die Messungen sollen im Spektralbereich zwischen etwa 7.000 und 700 Wellenzahlen (entsprechend 1,5 bis 15 µm) durchgeführt werden. Als Material für die Lichtleitfasern wird Chalcogenid-Glas vorgeschlagen.

Ein weiteres Beispiel für eine Publikation, die sich mit der ATR-Spektroskopie zur in vivo-Analyse von Körperbestandteilen, insbesondere Glucose, befaßt, ist

### 5) WO 91/18548.

Darin sind mehrere Ausführungsformen einer sogenannten ATR-Nadel dargestellt. Mit diesem Begriff werden Lichtleiter bezeichnet, die starr und an ihrem Unterende spitz derartig ausgebildet sind, daß sie in die menschliche Haut eingestochen werden können. Bei einer Ausführungsform ist vorgesehen, daß der ATR-Lichtleiter zusammen mit einer daneben verlaufenden metallenen Kanüle, die für die Infusion von Flüssigkeiten dient, in die Haut eingeführt wird. Bei einer anderen Weiterbildung ist die ATR-Nadel an ihrer Spitze mit einer Membran überzogen.

Ein anderes Meßprinzip, nämlich die Messung des Brechungsindex wird zur Messung von Glucose in Blut empfohlen in

### 6) WO 90/01697.

Auf Basis dieses Standes der Technik liegt der Erfindung die Aufgabe zugrunde, eine verbesserte Analysevorrichtung zur Bestimmung eines Analyten in vivo im Körper eines Patienten zur Verfügung zu stellen.

Die Aufgabe wird gelöst durch eine Analysevorrichtung gemäß Anspruch 1.

Im Rahmen der Erfindung wurde festgestellt, daß es im Gegensatz zu der in der Publikation 4) gegebenen Empfehlung vorteilhaft ist, wenn die Messung nicht auf eine Schleife an der Spitze der Kanüle konzentriert wird, sondern ein längerer Meßabschnitt von vorzugsweise mindestens 2 mm, besonders bevorzugt zwischen 3 mm und 10 mm Länge innerhalb einer über die Länge dieses Meßabschnittes penetrierbaren Kanüle zur Verfügung steht. Das Meßmedium ist dabei nicht das Blut in einer Ader, sondern die interstitielle Flüssigkeit im Hautgewebe, vorzugsweise im subcutanen Hautgewebe. Dabei wird eine verbesserte Genauigkeit und Empfindlichkeit erreicht, unter anderem weil im Rahmen der Erfindung festgestellt wurde, daß bei einer eng lokalisierten Messung ein hohes Risiko von Meßfehlern durch lokale Störungen sowohl hinsichtlich der Meßsonde, als auch hinsichtlich des umgebenden Hautgewebes besteht. Im Gegensatz zu den FOCS basiert die Analyse auf einer unmittelbaren reagenzienfreien Wechselwirkung des in der Lichtleitfaser entlang des Meßabschnittes transportierten Lichts mit der Probenflüssigkeit.

Die Kanüle ist in dem Meßabschnitt für die interstitielle Flüssigkeit zumindest insoweit penetrierbar, daß der Analyt in dem Meßabschnitt die für die Analyse erforderliche Wechselwirkung mit dem in der Lichtleitfaser transportierten Licht macht. Im Rahmen der Erfindung wurde festgestellt, daß bei den diskutierten sehr kleinen Abmessungen der Kanüle der Diffusionsaustausch des Analyten zwischen der die Kanüle umgebenden interstitiellen Flüssigkeit und der Oberfläche der Lichtleitfaser in dem Meßabschnitt so schnell erfolgt, daß die physiologische Änderung wichtiger Analyten, insbesondere der Glucose, mit hoher Genauigkeit verfolgt werden kann. Hierauf muß die Penetrierbarkeit der Kanüle abgestimmt sein. In dem bevorzugten Fall einer metallischen Kanüle wird diese Penetrierbarkeit durch eine entsprechende Perforation gewährleistet.

Grundsätzlich ist als Material für die Kanüle neben Metall auch ein hinreichend steifer Kunststoff geeignet. Dabei kann die erforderliche Penetrierbarkeit aus der Struktur des Kunststoffmaterials selbst resultieren, d.h. es kann ein Material verwendet werden, das auch ohne nachträglich angebrachte Löcher allein aufgrund seines Materials und des Herstellungsprozesses für die Analytmoleküle penetrierbar ist.

Die Ziele der Erfindung, vor allem eine gute Meßgenauigkeit und Verträglichkeit für den Patienten bei einer kontinuierlichen Messung über einen längeren Zeitraum (mindestens einen Tag, vorzugsweise mindestens drei Tage), können noch besser erreicht werden, wenn die nachfolgenden, bevorzugten Maßnahmen einzeln oder in Kombination miteinander angewendet werden.

Als Meßprinzip dient vorzugsweise ein Verfahren, bei dem die Wechselwirkung zwischen dem Licht und dem Analyten in dem Meßabschnitt auf dem Eindringen eines evaneszenten Feldes in die Flüssigkeit basiert, insbesondere die ATR-Spektroskopie. Die für die Gegenwart des Analyten charakteristische Veränderung des in der Lichtleitfaser transportierten Lichts ist dabei dessen wellenabhängige Schwächung in dem Meßabschnitt. Hinsichtlich der dabei üblichen Meß- und Auswerteverfahren kann in vollem Umfang auf die einschlägige Literatur, insbesondere auf die oben zitierten Publikationen verwiesen werden.

Die Wellenlänge des Meßlichts liegt bevorzugt im Bereich des mittleren Infrarot (MIR), insbesondere zwischen etwa 7 µm und 13 *µ*m. Dieser Wellenlängenbereich ist insbesondere für die Analyse von Glucose als Analyt geeignet.

Das Material der Lichtleitfaser soll in dem Spektralbereich des Meßlichtes möglichst transparent sein. Für die Zwecke der Erfindung ist insbesondere eine Silberhalogenid-Verbindung, insbesondere AgCl, AgBr oder Gemische davon, geeignet. Besonders bevorzugt sind Gemische mit einem überwiegenden Anteil an AgBr. Diese Materialien haben in dem angesprochenen Spektralbereich eine sehr geringe Absorption und können in Form von sehr dünnen elastischen Fasern hergestellt werden. Ein potentielles Problem bei der Verwendung im Kontakt mit Körperflüssigkeiten besteht darin, daß diese stets erhebliche Konzentrationen an Ionen enthalten und deshalb auf Silberhalogenid-Verbindungen korrosiv wirken. Im Rahmen der Erfindung wurde jedoch festgestellt, daß Silberhalogenid-Fasern, insbesondere in dem erwähnten Bereich von Lichtwellenlängen, ohne zusätzliche Schutzmaßnahmen in unmittelbarem Kontakt zu der interstitiellen Flüssigkeit für einen Zeitraum von mehreren Tagen ohne ein ihre Funktion gefährdendes Maß an Korrosion eingesetzt werden können.

Die Verwendung von Silberhalogenid-Fasern für nicht-medizinische Analysen ist beispielsweise aus folgenden Literaturstellen bekannt:

### 7) R. Göbel et al.: "Enhancing the Sensitivity of Chemical Sensors for Chlorinated Hydrocarbons in Water by the Use of Tapered Silver Halide Fibers and Tunable Diode Lasers", Applied Spectroscopy, 1995, 1174 bis 1177

### 8) J.F. Kastner et al.: "Optimizing the optics for Evanescent Wave Analysis With Laser Diodes (EWALD) for monitoring chlorinated hydrocarbons in water", SPIE Vol. 2783 (1996), 294 bis 306

### 9) DE 40 38 354 C2

Als weiteres, wenn auch weniger bevorzugtes, Material für die Lichtleitfaser kommt Chalcogenid-Glas in Betracht.

Nach neuesten Erkenntnissen läßt sich auch synthetischer Diamant in Form einer geeigneten Faser herstellen. Für die Zwecke der vorliegenden Erfindung genügt ein relativ kurzes, sehr dünnes Stück des Lichtleitfaser-Materials. Im Falle von Diamant hat die Faser bevorzugt aus Herstellungsgründen einen quadratischen oder rechteckigen Querschnitt. Das Material des synthetischen Diamanten ist bevorzugt durch schichtbildende Ablagerung aus der Gasphase, insbesondere durch "Chemical Vapor Deposition" (CVD) hergestellt. Dabei läßt sich aus der gebildeten Schicht eine dünne "Nadel" herausarbeiten, wie weiter unten noch näher erläutert wird. Die optischen Eigenschaften von Diamant im hier in Rede stehenden infraroten Wellenlängenbereich des Lichts sind zwar weniger gut als die der vorstehend genannten Fasermaterialien. Die Transmission ist jedoch zur Messung ausreichend. Vorteilhaft ist vor allem die hohe Korrosionsfestigkeit des Diamamtmaterials auch in salzhaltigen Lösungen. Nähere Einzelheiten sind der am 9. August 1997 eingereichten deutschen Patentanmeldung 19734617.0 "Vorrichtungen zum Untersuchen einer Probensubstanz mittels abgeschwächter Totalreflexion" zu entnehmen, deren Inhalt durch Bezugnahme zum Bestandteil der vorliegenden Anmeldung gemacht wird.

Weiterhin sind Germanium und Silicium als Material für den Lichtleiter geeignet. Diese Materialien haben bei hoher Reinheit gute Transmissionseigenschaften für IR-Licht (beispielsweise werden Ge-Objektive für IR-Kameras verwendet). Da die Mikromechanik für die Verarbeitung dieser Materialien hoch entwickelt ist, lassen sich die im Rahmen der Erfindung erforderlichen dünnen Nadeln gut herstellen. Zwar verursacht ihr hoher Brechungsindex Probleme bei der Einkopplung des Lichts. Der damit verbundene Intensitätsverlust ist jedoch vertretbar.

Im Rahmen der Erfindung muß die Lichtleitfaser allgemein keinen runden Querschnitt haben. Der Begriff "Faser" ist in dem Sinn zu verstehen, daß es sich um ein Stück Lichtleitendes Material mit einer Länge handelt, die der zum Einstechen in die Haut erforderlichen Länge der Kanüle (mindestens etwa 3 mm) entspricht und dessen Querschnitt im Verhältnis zu der Länge sehr klein ist. Bevorzugt sollte die Kanüle einen Außendurchmesser von maximal 0,8 mm, besonders bevorzugt höchstens 0,5 mm oder sogar nur 0,3 mm haben. Bei einer Wandstärke von 0,05 mm resultiert hieraus ein Innenquerschnitt mit einem Durchmesser von 0,7 mm beziehungsweise 0,5 mm und 0,2 mm. Der Faserquerschnitt muß so sein, daß er in dieses kleine Lumen der Kanüle paßt, wobei im Fall einer nicht runden Faser auch die Kanüle vorzugsweise eine entsprechende nichtrunde Querschnittsgestaltung hat.

Weitere bevorzugte Ausführungsformen, die anhand der in den Figuren dargestellten Ausführungsbeispiele näher erläutert werden, sehen folgende Merkmale vor:
- Der Querschnitt der Lichtleitfaser im Meßabschnitt ist nicht durchgängig gleich, sondern variiert. Es findet also ein- oder mehrfach ein Übergang von einem größeren Lichtleitfaser-Querschnitt zu einem kleineren Lichtleitfaser-Querschnitt statt. Durch diese in anderem Zusammenhang in den vorstehend genannten Publikationen bereits beschriebene Maßnahme wird die Zahl der Reflexionen in der Lichtleitfaser und infolgedessen die Empfindlichkeit der Messung erhöht.
- Die Lichtleitfaser ist von einer semipermeablen Membran derartig umhüllt, daß die interstitielle Flüssigkeit in dem Meßabschnitt nur durch die Membran an die Oberfläche der Lichtleitfaser gelangen kann. Die semipermeable Membran hat dabei eine Ausschlußgrenze für große Moleküle mit einer Molekülgröße von mehr als 5.000 Da, bevorzugt für Moleküle mit einer Molekülgröße von mehr als 1.000 Da. Die Durchlaßgrenze soll möglichst scharf und die Durchtrittsrate für kleinere Moleküle möglichst hoch sein. Durch die Membran wird bei gegebenem meßtechnischem Aufwand die Genauigkeit erhöht bzw. es ist möglich, eine gewünschte Meßgenauigkeit mit reduziertem Aufwand zu erreichen. Proteinablagerungen auf der Oberfläche des Lichtleiters und andere Störeinflüsse der größeren Moleküle werden vermieden. Beispielsweise ist es möglich, die Anzahl der für die Auswertung notwendigen Wellenlängen zu reduzieren. Darüber hinaus reduziert die semipermeable Membran die Abstoßungsreaktion des Körpers gegen die in die Haut eingestochene Sonde. Hieraus resultiert eine Verlängerung der erreichbaren Aufenthaltsdauer im Körper. Als Material der Membran kommen insbesondere Polysulfon-Polyamid, Polyethylen, Polycarbonat und Cellulose in Betracht.
- Die Lichtleitfaser ist in dem Meßbereich mit einer Beschichtung versehen, die mehrere Aufgaben erfüllen kann. Zum einen kann sie als Schutz gegen Korrosion der Faser dienen. Zum zweiten kann sie einen Abstandshälter bilden, um einen unmittelbaren Kontakt zwischen der Lichtleitfaser und einer sie umhüllenden semipermeablen Membran zu verhindern. Drittens kann ein Material für die Beschichtung gewählt werden, welches zu einer Anreicherung des Analyten an der Oberfläche der Lichtleitfaser führt.

Die Erfindung wird nachfolgend anhand von in den Figuren schematisch dargestellten Ausführungsbeispielen weiter erläutert; es zeigen:
- Fig. 1: eine erfindungsgemäße Analysevorrichtung in perspektivischer Darstellung,
- Fig. 2: eine perspektivische Darstellung der Elektronikeinheit der Vorrichtung von Fig. 1,
- Fig. 3: eine Querschnittdarstellung des Sondenkopfes der Vorrichtung von Fig. 1,
- Fig. 4: eine Prinzipdarstellung der Einstrahlungs- und Detektionsmittel bei einer ersten Ausführungsform der Erfindung,
- Fig. 5: eine alternative Ausführungsform der einstechbaren Meßsonde,
- Fig. 6: eine weitere alternative Ausführungsform der einstechbaren Meßsonde,
- Fig. 7: eine Prinzipdarstellung von für die Meßsonde nach Fig. 6 geeigneten Lichteinstrahlungs- und Detektionsmitteln,
- Fig. 8: einen Querschnitt durch eine Meßsonde ähnlich Fig. 6,
- Fig. 9: einen Längsschnitt durch den oberen (proximalen) Teil einer Meßsonde ähnlich Fig. 6,
- Fig. 10: eine perspektivische Prinzipdarstellung eines Teils einer weiteren abgewandelten Ausführungsform einer Meßsonde,
- Fig. 11: einen Querschnitt einer ersten Variante ähnlich der Ausführungsform nach Fig. 10,
- Fig. 12: einen Querschnitt einer zweiten Variante ähnlich der Ausführungsform nach Fig. 10,
- Fig. 13: eine perspektivische Teilansicht einer ersten Ausführungsform einer Anordnung zum Ein- oder Auskoppeln von Licht in eine auf einem Substrat befindliche Lichtleitfaser,
- Fig. 14: eine Seitenansicht einer zu Figur 13 abgewandelten Ausführungsform,
- Fig. 15: eine Vorderansicht zu Figur 14,
- Fig. 16: eine perspektivische Teilansicht einer dritten abgewandelten Ausführungsform zu Figur 13,
- Fig. 17: eine Aufsicht auf eine vierte abgewandelte Ausführungsform zu Figur 13,
- Fig. 18: eine Seitenansicht zu Figur 17.

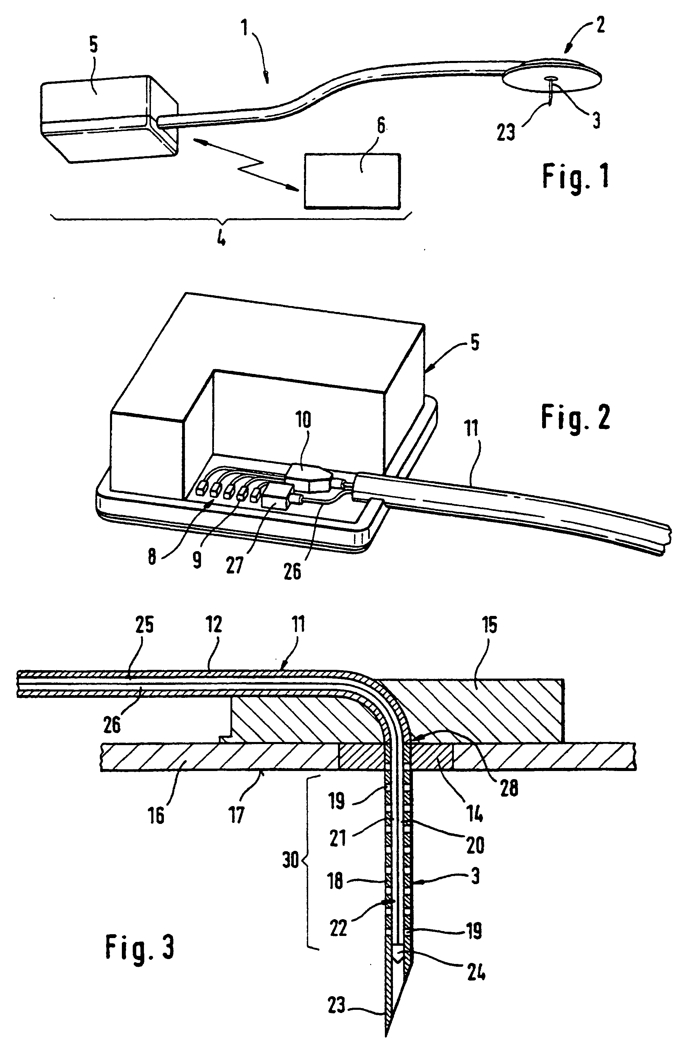

Die in Figur 1 dargestellte Analysevorrichtung 1 besteht im wesentlichen aus einem Sondenkopf 2 mit einer in die Haut einstechbaren Meßsonde (Einstechsonde) 3 und einer Meß- und Auswerteeinheit 4. Die Meß- und Auswerteeinheit 4 hat im dargestellten bevorzugten. Fall zwei räumlich getrennte Teile, nämlich eine gemeinsam mit dem Sonden-kopf 2 am Körper des Patienten tragbare Elektronikeinheit 5, die vorzugsweise nur diejenigen elektronischen Elemente enthält, die erforderlich sind, um das Meßlicht in die Sonde 3 einzuspeisen und an dem aus der Sonde 3 zurückkommenden Meßlicht eine für die Konzentration des Analyten charakteristische Veränderung zu messen. Die dabei resultierenden Meßsignale werden in der Elektronik-einheit 5 gespeichert und zur Auswertung - vorzugsweise drahtlos - an eine zentrale Auswerteelektronik 6 übertragen, die der zweite Bestandteil der Meß- und Auswerteeinheit 4 ist und elektronische Mittel zum Empfangen der Meßsignale und zur Weiterverarbeitung in der jeweils benötigten Art und Weise aufweist.

Einzelheiten der Funktion der Meß- und Auswerteeinheit und der Verteilung dieser Funktion auf die beiden dargestellten Komponenten 5 und 6 hängen von dem jeweiligen Einzelfall ab. Beispielsweise kann es zweckmäßig sein, daß die Elektronikeinheit 5 ein ausreichendes Maß an Intelligenz enthält, um Konzentrationswerte des zu bestimmenden Analyten zu ermitteln und mittels eines Display anzuzeigen..Die Auswerteelektronik 6 erfüllt langfristigere Aufgaben, insbesondere die längerfristige Speicherung der Meßdaten, die Darstellung von Kurven etc. Wenn die Analysevorrichtung zur Bestimmung der Blutglucose bei Diabetikern ausgebildet ist, kann es beispielsweise zweckmäßig sein, die Blutglucosewerte laufend an der Elektronikeinheit anzuzeigen und beim Unter- bzw. Überschreiten bestimmter Grenzwerte ein Alarmsignal auszulösen. Die Auswerteelektronik dient dann dazu, Daten für die Verwendung durch den Arzt zu speichern und möglicherweise Insulindosierungen für die Therapie des Patienten zu berechnen.

Nähere Einzelheiten der Elektronikeinheit 5 und der Meßsonde 2 sind in den Figuren 2 und 3 zu erkennen. In der Elektronikeinheit 5, die in Figur 2 mit abgenommenem Deckel dargestellt ist, befindet sich mindestens eine Lichtquelle 8 zur Erzeugung des Meßlichts. Im dargestellten Fall sind fünf Halbleiterlichtquellen 9 (Leuchtdioden oder Laserdioden) vorgesehen, deren Licht durch eine Strahl-Zusammenfassungseinheit (beam combiner) zusammengefaßt wird. Der resultierende Lichtstrahl wird in ein faseroptisches Kabel 11 eingespeist, durch das die Elektronikeinheit 5 mit der Sonde 2 verbunden ist.

In dem Sondenkopf 2 ist die Einstechsonde 3 mit Hilfe von zwei Haltescheiben 14 und 15 gelagert (Fig.3). Die obere Haltescheibe 15 dient zugleich als Zugentlastung und Führung für das faseroptische Kabel 11. Zur Befestigung an der Haut ist eine Hautkontaktscheibe 16 vorgesehen, die beispielsweise eine klebende Unterseite 17 haben kann, um die Sonde 3 an der Hautoberfläche zu befestigen. Statt der dargestellten senkrechten Orientierung zwischen der Einstechsonde 2 und der Hautkontaktscheibe 16 kann auch eine Anordnung vorgesehen sein, bei der die Einstechsonde unter einem kleineren Winkel als 90° in die Haut eindringt, wobei Winkel zwischen 30° und 60° zur Hautoberfläche bevorzugt sind. Dadurch kann eine relativ lange Einstechsonde verwendet und dennoch die Eindringtiefe unterhalb einer aus physiologischen Gründen gewünschten Obergrenze gehalten werden.

Die Einstechsonde 3 besteht im wesentlichen aus einer mit Löchern 19 perforierten Kanüle 18 aus einem physiologisch unbedenklichen Material (zum Beispiel Edelstahl), in der zwei Faserstrecken 20 und 21 einer Lichtleitfaser 22 parallel verlaufen sowie einem im Bereich des distalen Endes 23 der Kanüle 18 angeordneten prismatischen Reflektor 24.

Der Reflektor 24 ist so an die distalen Enden der Faserstrecken 20 und 21 angekoppelt, daß über eine der Fasern (einleitende Faserstrecke 20) eingekoppeltes Licht in die andere Faser (rückleitende Faserstrecke 21) zurückreflektiert wird.

Bei der dargestellten Ausführungsform verlaufen in dem faseroptischen Kabel 11 zwei Lichtleitfaserstrecken innerhalb einer flexiblen Umhüllung 12 parallel. Das Meßlicht wird von der Strahlzusammenfassungseinheit 10 in die erste (einleitende) Lichtleitfaserstrecke 25 des Kabels 11 eingekoppelt und weiter in die einleitende Faserstrecke 20 der Meßsonde 3 geleitet. Nach Reflexion an dem Reflektor 24 wird es durch die rückleitende Faserstrecke 21 der Meßsonde 3 und eine entsprechende rückleitende Faserstrecke 26 des faseroptischen Kabels 11 in die Elektronikeinheit 5 zurückgeleitet, wo das Meßlicht von einem Detektor 27 detektiert wird.

Bei der dargestellten bevorzugten Ausführungsform sind die einleitenden Faserstrecken 25 und 20 sowie die rückleitenden Faserstrecken 26 und 21 der Sonde 3 und des faseroptischen Kabels 11 jeweils einstückig ausgebildet, d.h. sie bestehen jeweils aus einer durchgehenden Faser eines einheitlichen Fasermaterials. Dies ist im Hinblick auf eine leichte Herstellung und geringe Intensitätsverluste bevorzugt.

Es besteht jedoch auch die Möglichkeit, die in der Meßsonde 3 verlaufenden Faserstrecken aus einem anderen Material als die außerhalb der Meßsonde (in dem faseroptischen Kabel 11) verlaufenden Faserstrecken herzustellen und das Licht im Bereich des proximalen Endes 28 der Kanüle 18 in die jeweils anschließende Faserstrecke einzukoppeln. Dies ist insbesondere dann sinnvoll, wenn technologische Schwierigkeiten bestehen, ein für die Gesamtstrecke 25,20 bzw. 26,21 ausreichend langes Stück aus dem Fasermaterial herzustellen, das für die in der Sonde verlaufenden Strecken 20,21 der Lichtleitfaser 22 verwendet wird.

Die Perforationslöcher 19 erstrecken sich über einen Teilabschnitt der Kanüle 18. Die entsprechende Teillänge der in der Kanüle 18 verlaufenden Lichtleitfaser 22 (im dargestellten Fall beider Faserstrecken 21 und 22) wird als Meßabschnitt 30 bezeichnet. In dem Meßabschnitt 30 steht die Außenseite der Lichtleitfaser 22 in dem Hautgewebe über die Perforationslöcher 19 mit der die Kanüle 18 umgebenden interstitiellen Flüssigkeit in Kontakt. Um einen intensiven Austausch zu ermöglichen, ist die Kanüle 18 möglichst stark perforiert. Der Lochdurchmesser hängt wesentlich vom Verfahren der Herstellung der Löcher ab und kann in weiten Grenzen variieren, er sollte bevorzugt zwischen etwa 1 µm und etwa 100 µm liegen. Die Löcher können insbesondere durch Laserbohrverfahren erzeugt werden. Der Flächenanteil der Perforationslöcher an der Oberfläche der Kanüle in dem Meßabschnitt 30 sollte ausreichend groß sein. Derzeit wird ein Porenanteil von mindestens 20%, bevorzugt mindestens 50% angestrebt.

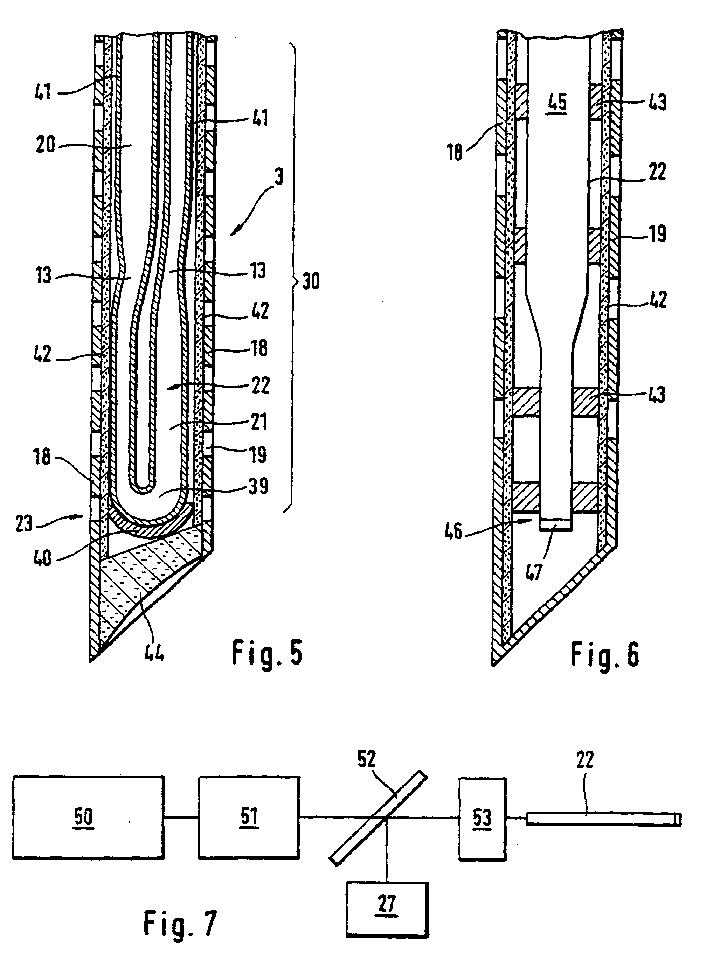

Die Kanüle 18 kann neben der Diagnose des Analyten zugleich dazu dienen, ein Arzneimittel (insbesondere Insulin) subkutan zu applizieren. In diesem Fall enthält das Gehäuse der Elektronikeinheit 5 eine nicht dargestellte Insulinpumpe und das faseroptische Kabel 11 einen nicht dargestellten Schlauch zum Transport des Arzneimittels in die Kanüle 18. Das Arzneimittel strömt in der Kanüle 18 an der Lichtleitfaser 22 vorbei und gelangt durch die Perforationslöcher 19 in das Gewebe. Dadurch wird der Kontakt zwischen der interstitiellen Flüssigkeit und der Oberfläche der Lichtleitfaser unterbrochen. Dies kann zur Null-Kalibration der optischen Messung vorteilhaft genutzt werden. Auf dem gleichen Weg kann auch eine Spüllösung oder eine Kalibrations-Standardlösung zugeführt werden. Bei dieser Ausführungsform ist es vorteilhaft, wenn die Kanüle an ihrem distalen Ende offen ist, so daß die zugeführte Flüssigkeit hier austreten kann. Bei anderen Ausführungsformen ist jedoch eine am distalen Ende geschlossene Kanüle, wie sie in den Figuren 5 und 6 dargestellt ist, bevorzugt. In jedem Fall sollte der in distaler Richtung am meisten vorstehende Abschnitt der Lichtleitfaser 22 gegenüber dem distalen Ende 23 der Kanüle 18 zurücktreten, so daß die empfindliche Lichtleitfaser 22 vollständig von der Kanüle 18 umgeben und durch diese geschützt ist.

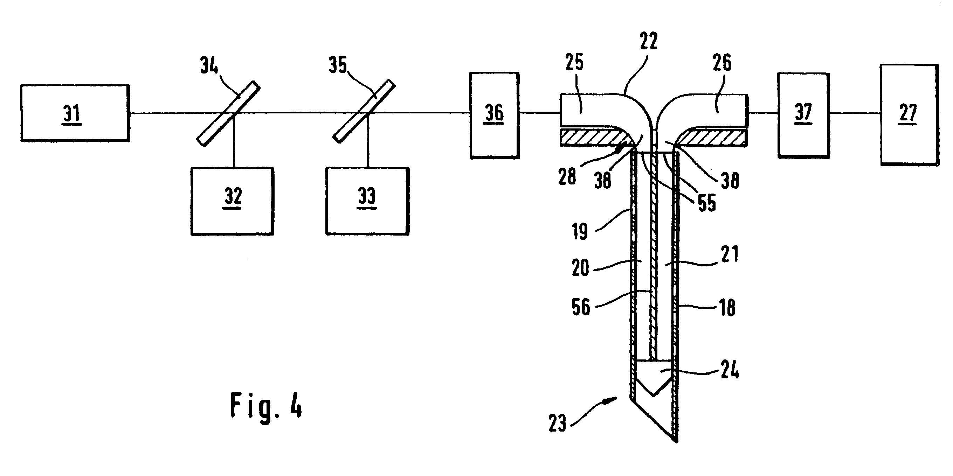

Als Lichtquelle für das Meßlicht ist eine Laserlichtquelle bevorzugt, insbesondere weil sie eine hohe spektrale Leuchtdichte ermöglicht und sich gut auf die Stirnflächen sehr dünner Lichtleitfasern fokussieren läßt. Laserlichtquellen sind monochromatisch und haben in der Regel eine feste unveränderliche Wellenlänge. In Figur 4 ist dargestellt, in welcher Weise bei der Erfindung eine spektroskopische Messung mit mehreren Laserlichtquellen unterschiedlicher Wellenlängen möglich ist.

Bei der dargestellten Ausführungsform sind für drei verschiedene Wellenlängen drei Laser 31 bis 33 vorgesehen. Der Ausgang des ersten Lasers 31 ist unmittelbar in die optische Achse der Anordnung gerichtet. Das Licht der beiden anderen Laser 32 und 33 wird mittels halbdurchlässiger Spiegel 34 und 35 in die gleiche optische Achse gelenkt. Zur Einkopplung in die einleitende Faserstrecke 25,20 ist eine Koppeloptik 36 vorgesehen. Eine zweite Koppeloptik 37 dient dazu, das über die ausleitenden Faserstrecken 21,26 zugeführte Meßlicht auf den Detektor 27 auszukoppeln. Die spektrale Empfindlichkeit des Detektors 27 ist hinreichend breitbandig, daß er sämtliche Wellenlängen der Laser 31 bis 33 detektieren kann.

Selbstverständlich können zur Messung mit mehr als drei Wellenlängen entsprechend mehr Laser verwendet werden. Bevorzugt kommen zur Messung im mittleren Infrarot Quantenkaskadenlaser zum Einsatz.

Eine weitere Besonderheit der in Figur 4 dargestellten Ausführungsform gegenüber Figur 3 besteht darin, daß vor dem Eintritt der Lichtleitfaser 22 in die Kanüle 18 ein Übergang von einer dickeren in dem Kabel 11 verlaufenden Faserstrecke 25 zu einer im Querschnitt dünneren Faserstrecke 20 in der Kanüle 18 stattfindet. Entsprechend findet auf der Austrittsseite ein Übergang von einer dünneren Faserstrecke 21 in der Kanüle 18 zu einer dickeren Faserstrecke 26 in dem Kabel 11 statt. In Figur 4 ist lediglich aus Gründen der Anschaulichkeit dargestellt, daß die Faserstrecken 25 und 26 in entgegengesetzte Richtung verlaufen. In aller Regel wird es zweckmäßiger sein, sie durch ein einziges Kabel 11 zu führen.

Die in Figur 4 dargestellte Verjüngung 38 der Lichtleitfaser 22 vor dem Eintritt in die Kanüle 18 hat den Vorteil, daß in dem faseroptischen Kabel eine verhältnismäßig dicke Lichtleitfaser verwendet werden kann, die sich durch eine bessere mechanische Stabilität und geringere optische Verluste auszeichnet. Darüber hinaus.hat es sich nicht nur wegen des geringeren Schmerzes bei einer dünnen Kanüle 18, sondern aus Gründen der Meßempfindlichkeit als vorteilhaft herausgestellt, wenn die Lichtleitfaser in der Kanüle 18 einen sehr kleinen Querschnitt (entsprechend einem Durchmesser von weniger als 0,2 mm) hat.

In der in Figur 4 dargestellten Ausführungsform mit der Verjüngung 38 ist es vorteilhaft, wenn - wie bereits oben erläutert - die Faserstrecken 20,21 getrennt von den Faserstrecken 25,26 hergestellt sind und möglicherweise aus einem anderen Material bestehen. Die Überkopplungsstellen im Bereich des proximalen Endes 28 sind in Figur 4 mit 55 bezeichnet. Die Faserstrecken 20,21 in der Kanüle 18 sind durch einen beidseitig verspiegelten Metallstreifen 56 getrennt. Dadurch wird optisches Übersprechen verhindert. Außerdem können die zu diesem Zweck bevorzugt im Querschnitt halbkreisförmig ausgebildeten Faserstrecken 20,21 bei der Montage des Sensors zunächst an dem Trennstreifen 56 befestigt und dann gemeinsam in die Kanüle eingeführt werden.

In Figur 5 ist eine Einstechsonde 3 dargestellt, bei der ebenso wie bei Figur 3 in der Kanüle 18 zwei Lichtleitfaserstrecken 20,21 parallel verlaufen, wobei das Licht durch eine einleitende Faserstrecke 20 in Richtung auf das distale Ende 23 der Kanüle 18 transportiert wird. Ebenso wie bei Figur 3 findet im Bereich des distalen Endes 23 der Kanüle 18 eine Umlenkung in die Gegenrichtung statt und das Licht wird durch die ausleitende Faserstrecke 21 aus der Kanüle 18 ausgeleitet. Die in Figur 5 dargestellte Ausführungsform unterscheidet sich von der Ausführungsform gemäß Figur 3 hinsichtlich der nachfolgenden Merkmale.

Die Umlenkung des Lichts im Bereich des distalen Endes 23 der Kanüle 18 wird hier durch eine enge Umlenkschleife 39 einer durchgehenden Lichtleitfaser 22 erreicht. Im Gegensatz zu Zitat 4) wird der Bereich der Umlenkschleife 39 nicht zur Messung benutzt. Im Gegenteil wird durch eine verspiegelte Kappe 40 gewährleistet, daß das Licht mit möglichst geringen Reflexionsverlusten und ohne Auskopplung umgelenkt wird.

Die Lichtleitfaserstrecken 20 und 21 weisen jeweils eine Verjüngungsstelle 13 auf, an der der Querschnitt der Lichtleitfaser von einem größeren Wert auf einen kleineren Wert übergeht, um dadurch, wie beschrieben, die Anzahl der Reflexionen und die Empfindlichkeit der Messung zu erhöhen. Statt der dargestellten relativ schnellen Übergänge, kann auch eine langsame Verjüngung über den gesamten Meßabschnitt 30 vorteilhaft sein. Es ist auch möglich, in jeder in dem Meßabschnitt 30 verlaufenden Lichtleitfaserstrecke mehrere Bereiche vorzusehen, in denen der Querschnitt der Lichtleitfaser variiert.

Die Lichtleitfaser 22 in Figur 5 ist mit einer Beschichtung 41 versehen, die einerseits die Messung nicht stören darf, andererseits eine oder mehrere der oben erwähnten Aufgaben erfüllt. In Betracht kommen insbesondere folgende Typen.von Beschichtungsmaterialien.

Die Beschichtung kann aus einer sehr dünnen (beispielsweise aufgedampften) Metallschicht bestehen. Das Metall (bevorzugt Edelmetall, vor allem Silber) bildet einen Schutz der Lichtleitfaser 22 gegen Korrosion. Außerdem ist es als Abstandshalter für eine die Lichtleitfaser 22 umgebende Membran 42 geeignet. Da der Austritt evaneszenter Wellen schon durch eine sehr dünne metallische Beschichtung erheblich beeinträchtigt wird, sollte sie im Falle von ATR-Messungen unterbrochen sein, so daß auf einem erheblichen Teil der Oberfläche ein unmittelbarer Kontakt zwischen der interstitiellen Flüssigkeit und der Oberfläche der Lichtleitfaser möglich ist. In Verbindung mit einer dünnen metallischen Beschichtung ist jedoch auch ein anderer Wechselwirkungsmechanismus zwischen dem in der Lichtleitfaser 22 transportierten Licht und der interstitiellen Flüssigkeit, nämlich über Oberflächenplasmonen, möglich.

Alternativ kann es vorteilhaft sein, die Lichtleitfaser 22 (zumindest in dem Meßabschnitt 30) mit einer Beschichtung aus einem polymeren Material zu versehen. Das verwendete Polymer darf in dem Spektralbereich des Meßlichts nur eine geringe Absorption haben. Auch die polymere Beschichtung dient dem Schutz der Faser vor Korrosion und verhindert als Abstandshalter eine unmittelbare Berührung zwischen der Membranhülle 42 und der Lichtleitfaser 22. Nach derzeitigem Kenntnisstand kommen als geeignete Polymere insbesondere folgende Materialien in Betracht: Polytetafluorethylen, Polyisobutylen, Polycarbonat.

Besonders bevorzugt ist eine polymere Beschichtung, die analytanreichernde Eigenschaften hat. Für nichtmedizinische ATR-Meßverfahren ist dies in den Zitaten 8) und 9) beschrieben. Eine Beschichtung, die diese Eigenschaften hat, muß jeweils für den gewünschten Analyten experimentell gefunden werden.

Eine weitere Besonderheit der in Figur 5 dargestellten Ausführungsform besteht darin, daß eine die Lichtleitfaser 22 in dem Meßabschnitt 30 umhüllende Membran 42 vorgesehen ist. Wie bereits erwähnt, verhindert die Membran den Zutritt hochmolekularer Substanzen an die Oberfläche der Lichtleitfaser 22, wodurch eine verbesserte Meßgenauigkeit mit verhältnismäßig geringem meßtechnischem Aufwand erreicht werden kann. Selbstverständlich muß dabei durch geeignete Abdichtungsmaßnahmen erreicht werden, daß die interstitielle Flüssigkeit nicht durch verbleibende Spalten in einem praktisch störenden Umfang an die Lichtleitfaser 22 gelangen kann. Im dargestellten Fall ist beispielsweise die Kanüle 18 durch einen Tropfen 44 Epoxidharz verschlossen, der gleichzeitig die untere Abdichtung der Membran 42 sicherstellt.

Geeignete Membranmaterialien, die die mechanischen, chemischen und Dialyseeigenschaften für die vorliegende Anwendung haben, sind von sogenannten Mikrodialyseverfahren bekannt. Hierzu kann insbesondere verwiesen werden auf:

### 10) US-Patent 4,694,832 und die darin zitierten Literaturstellen

Besonders geeignete Membranmaterialien wurden weiter oben bereits erwähnt.

Im Rahmen der Erfindung wurde festgestellt, daß die vorteilhafte Wirkung einer die Lichtleitfaser 22 umhüllenden Membran 42 dadurch negativ beeinflußt werden kann, daß die Membran die Lichtleitfaser 22 unmittelbar berührt. Der Lichttransport des Meßlichts in der Lichtleitfaser 22 kann dadurch in einer für die Meßgenauigkeit sehr ungünstigen Weise beeinträchtigt werden. Deswegen ist es im Rahmen der vorliegenden Erfindung besonders bevorzugt, daß die Membran von der äußeren Oberfläche der Lichtleitfaser 22 im wesentlichen beabstandet ist. "Im wesentlichen beabstandet" ist dabei dahingehend zu verstehen, daß eventuell verbleibende Berührungsflächen zwischen der Membran und der äußeren Oberfläche der Lichtleitfaser 22 so klein sind, daß die Meßgenauigkeit hierdurch nicht in einem störenden Umfang beeinträchtigt wird. Die Berührungsfläche sollte jedenfalls weniger als 50%, bevorzugt weniger als 20% und besonders bevorzugt weniger als 10% der Oberfläche der Lichtleitfaser 22 in dem Meßabschnitt 30 betragen. Auch die Größe des Abstandes zwischen der Lichtleitfaser 22 und der Membran (dort wo sie beabstandet sind) ist für die Meßgenauigkeit bedeutsam. Mindestens sollte er dem zweifachen, bevorzugt mindestens dem dreifachen der Wellenlänge des Meßlichts entsprechen. Der maximale Abstand resultiert aus den Abmessungen der Bauteile und dem Erfordernis eines ausreichend schnellen Diffusionsaustauschs. Er liegt in der Praxis unter 100 *µ*m. Als Abstandshalter kann - wie in Figur 5 dargestellt - eine Beschichtung 41 der Lichtleitfaser 22 dienen. Der Abstand zwischen der Membran 42 und der Lichtleitfaser 22 ist nicht erforderlich, wenn die Membran in dem Spektralbereich des Meßlichtes keine störende optische Absorption hat.

In Figur 6 ist eine alternative Möglichkeit zur Erzeugung eines Abstands zwischen der Lichtleitfaser 22 und der Membran 42 dargestellt. Die Membran 42 ist hier auf die innere Oberfläche der Kanüle 18 beschichtet. Die Lichtleitfaser 22 ist mit Abstandsringen 43, welche bevorzugt aus einem im Infrarotbereich nicht oder nur schwach absorbierenden Material bestehen, in der Kanüle 18 fixiert. Alternativ - wenn auch nach derzeitigem Kenntnisstand weniger bevorzugt, besteht auch die Möglichkeit, die Membran auf der äußeren Oberfläche der Kanüle 18 aufzubringen, wobei der Abstand zu der Oberfläche der Lichtleitfaser 22 dann selbstverständlich durch die Wand der Kanüle 18 gewährleistet wird.

Eine weitere Besonderheit der in Figur 6 dargestellten Ausführungsform besteht darin, daß innerhalb der in diesem Fall unten geschlossenen Kanüle 18 in dem.Meßabschnitt 30 nur eine Lichtleitfaserstrecke 45 verläuft, an deren distalem Ende 46 eine Verspiegelung 47 beispielsweise aus Gold vorgesehen ist, durch das das Licht in die gleiche Lichtleitfaserstrecke 45 zurückreflektiert wird.

Bei der in Figur 6 dargestellten Ausführungsform ist es erforderlich, das Meßlicht in geeigneter Weise in die einzige Lichtleitfaserstrecke 45 ein- und wieder aus dieser auszukoppeln. Eine Möglichkeit hierzu ist in Figur 7 schematisch dargestellt. Aus dem Licht einer breitbandig emittierenden Lichtquelle 50 wird mittels eines Bandpaß-Spektralfilters 51 der gewünschte Wellenlängenbereich selektiert. Das resultierende Licht fällt durch einen Strahlteiler 52 über eine Koppeloptik 53 in die Lichtleitfaser 22. Nach Reflexion an dem Reflektor 47 fällt das Licht durch die Koppeloptik 53 auf den Strahlteiler 52 zurück und wird von diesem in den Detektor 27 reflektiert. Statt der breitbandigen Lichtquelle 50 kann auch in diesem Fall eine Anordnung mehrerer Laser (wie bei Figur 4) eingesetzt werden.

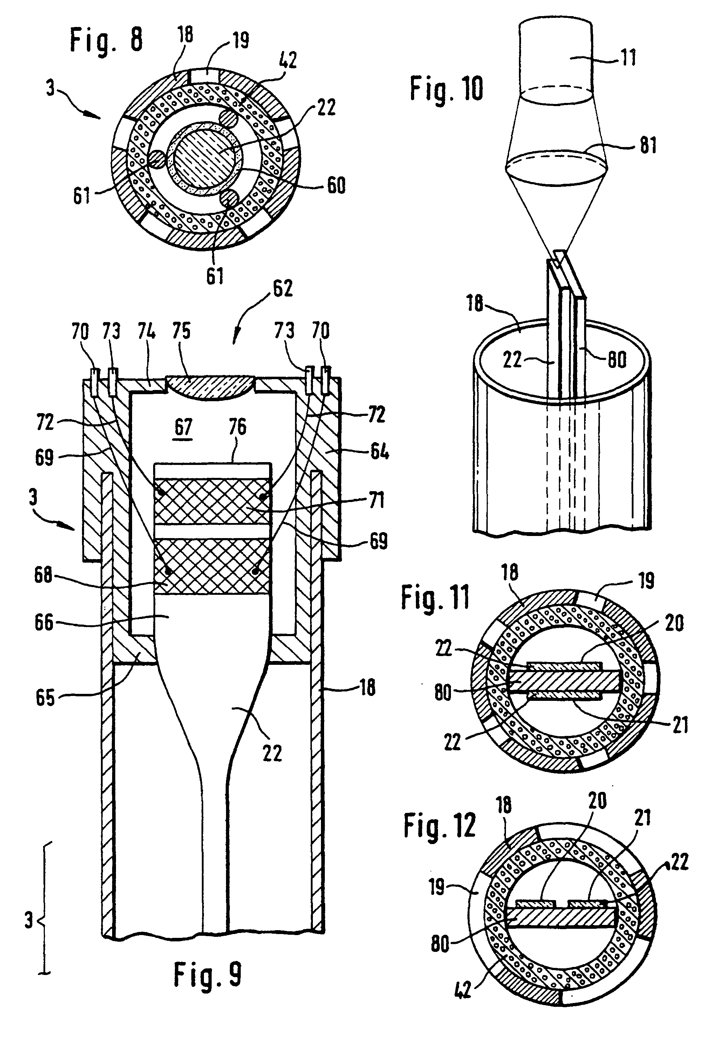

Figur 8 zeigt einen Querschnitt durch eine Einstechsonde 3, die ähnlich Figur 6 aufgebaut ist, jedoch weist die Lichtleitfaser 22 eine Beschichtung 60 auf, die aus Diamant bzw. diamantartigem Kohlenstoff (diamond like carbon, DLC) besteht. Die Beschichtung umgibt die Lichtleitfaser über die gesamte Länge des Meßabschnitts 30 vorzugsweise vollständig. Eine solche Diamantbeschichtung stellt einen wirksamen Schutz des Materials der Lichtleitfaser 22 gegen Korrosion dar. Außerdem ist sie vorteilhaft im Hinblick auf die Biokompatibilität. Sie ermöglicht sogar die Verwendung toxischer Substanzen, wie beispielsweise einer T1J-TlBr-Mischung für die Lichtleitfaser.

Vorzugsweise wird die Diamantbeschichtung 60 durch Abscheiden aus der Gasphase hergestellt, wobei ein Niedertemperatur-CVD-Prozeß besonders bevorzugt ist.

Die Beschichtung eines Lichtleiters mit einer Diamantschicht ist aus der WO 97/06426 bekannt. Gemäß dieser Literaturstelle soll die Diamantschicht stets einen kleineren Brechungsindex als der Lichtleiter haben. Dadurch soll sichergestellt werden, daß - ebenso wie bei dem im allgemeinen bei Lichtleitfasers weitgehend gebräuchlichen "Cladding" - die für den Lichttransport in der Lichtleitfaser erforderliche Totalreflexion nicht gefährdet wird. Im Gegensatz zu diesem Stand der Technik wird bei der vorliegenden Erfindung bevorzugt eine Beschichtung - insbesondere aber nicht ausschließlich aus Diamant - eingesetzt, deren Brechungsindex größer oder mindestens ebenso groß wie der der Lichtleitfaser ist. Wenn diese Schicht sehr dünn ist, geht die Totalreflexion nicht verloren. Vielmehr ergibt sich eine effektive Wechselwirkung des in der Lichtleitfaser transportierten Lichts über das umgebende evaneszente Feld mit der die Beschichtung umgebenden Flüssigkeit. Dieses Verhalten gilt insbesondere bei einer Ausführungsform mit einem Lichtleiter aus einem Silberhalogenid, der mit einer Diamantschicht beschichtet ist.

In Figur 8 ist eine weitere Möglichkeit dargestellt, einen Abstand zwischen der Lichtleitfaser 22 und der Membran 42 zu realisieren, nämlich mittels in Längsrichtung der Kanüle verlaufender Metalldrähte (vorzugsweise aus Gold).

Figur 9 zeigt eine vorteilhafte Gestaltung des proximalen Endes 62 einer Meßsonde 3. In dem dargestellten Fall ist eine trennbare (steckbare) Verbindung zwischen dem zur Lichtzuführüng dienenden faseroptischen Kabel 11 und der in der Kanüle verlaufenden Faserstrecke der Lichtleitfaser 22 vorgesehen. Ein insgesamt mit 64 bezeichnetes Verschlußstück ist auf das proximale Ende 66 der Kanüle 18, das man auch als Koppelende bezeichnen kann, aufsteckbar. Es weist einen Halterungskragen 65 auf, der die Lichtleitfaser 22 spielfrei und abdichtend umschließt. Zweckmäßigerweise ist deshalb das Verschlußstück 64 aus einem hinreichend elastischen Material gefertigt. Die Lichtleitfaser 22 ragt mit ihrem proximalen und verdickten Ende 66 in einen in dem Verschlußstück 64 ausgebildeten Koppelhohlraum 67 hinein, welcher so groß ausgestaltet ist, daß zwischen seinen Begrenzungswänden und dem darin befindlichen Abschnitt der Lichtleitfaser ein Freiraum vorhanden ist. An dem in den Koppelhohlraum 67 eindringenden Teil der Lichtleitfaser 22 ist ein Heizfilm 68, beispielsweise aus einer Nickel-Chrom-Legierung vorgesehen, der über Heizleitungen 69 mit Heißanschlüssen 70 verbunden ist, über die elektrische Leistung zugeführt werden kann.

Weiterhin ist an dem in den Koppelhohlraum 67 eindringenden Teil der Lichtleitfaser 22 ein Temperaturmeßfilm 71, beispielsweise in Form eines Pt-100-Schichtmeßwiderstandes, aufgebracht, der über Meßleitungen 72 mit Meßanschlüssen 73 verbunden ist. An der oberen Abschlußfläche des Verschlußstückes 64 ist ein Fixierkragen 74 ausgebildet, in den eine Koppellinse 75 zum Ein- und Auskoppeln von Licht in die proximale Stirnfläche 76 der Lichtleitfaser 22 eingefügt ist.

Diese Ausführungsform ist besonders vorteilhaft, wenn die Lichtleitfaser 22 aus Diamant besteht. Durch die sehr hohe Wärmeleitfähigkeit des Diamants kann trotz des sehr geringen Querschnitts der Lichtleitfaser 22 mittels des Heizfilms 68 und des Temperaturmeßfilms 71 eine wirksame Temperierung des Meßabschnittes der Lichtleitfaser 22 erfolgen, obwohl diese relativ weit von dem Meßabschnitt 30 entfernt am proximalen Ende der Lichtleitfaser 22 in dem Verschlußstück 64 angeordnet sind. Die genaue Temperaturmessung mittels des Temperaturmeßfilms 71 oder eines anderen Temperaturmeßelementes kann vorteilhaft bei der Auswertung der spektralen Messungen verwendet werden, um Temperatureinflüsse zu kompensieren.

Eine Lichtleitfaser 22 der gewünschten kleinen Abmessungen läßt sich vorzugsweise durch Herausarbeiten aus einer durch Ablagerung aus der Gasphase hergestellten dünnen Diamantschicht erzeugen. Die Herstellung von Diamantschichten mit Hilfe des CVD-Verfahrens ist bekannt und wird beispielsweise im Zusammenhang mit einem Enzym-Glucosesensor in der Publikation
C.E. Troupe et al. "Diamond-based glucose sensors" in Diamond and Related Materials, 1968, 575 bis 580 beschrieben: Um aus einer nach dem CVD-Verfahren auf einem Substrat erzeugten Schicht eine dünne nadelförmige freitragende Lichtleitfaser, wie sie in Figur 9 dargestellt ist, herauszuarbeiten, wird zweckmäßigerweise zunächst ihre Oberfläche glatt poliert. Danach wird das gewünschte seitliche Profil mittels eines Laser ausgeschnitten. Schließlich wird das Substrat, auf dem der CVD-Prozeß stattgefunden hat, durch Ätzen entfernt. Dabei sollten Verfahren verwendet werden, die möglichst glatte Oberflächen erzeugen.

Bei Verwendung eines Lichtleitfasermaterials, das als dünne Schicht auf einem Substrat hergestellt wird, wie insbesondere Diamant, Silizium und Germanium, kann es vorteilhaft sein, die Lichtleitfaser zusammen mit dem Substrat in die Kanüle zu integrieren. Eine solche Anordnung ist in Figur 10 stark schematisiert dargestellt. Auf einem im Querschnitt rechteckigen Substrat 80 befindet sich die im Querschnitt ebenfalls rechteckige Lichtleitfaser 22. Zur stirnseitigen Einkopplung des Lichts von einem faseroptischen Kabel 11 ist eine Freistrahl-Einkopplungsoptik 81 vorgesehen. Sie kann beispielsweise wie in Figur 9 durch ein Verschlußstück 64 mit Koppellinse 75 ausgebildet sein.

Die Kanüle 18 ist in Figur 10 in übertriebener Größe dargestellt. In Anbetracht der im Rahmen der Erfindung gewünschten extrem kleinen Abmessungen der Kanüle 18 erscheint es zunächst problematisch, in deren kleines Lumen mit einem Durchmesser von beispielsweise weniger als 0,5 mm nicht nur eine Lichtleitfaser, sondern zusätzlich eine Substratschicht zu integrieren. Die Kombination CVD-Lichtleitfaserschicht mit Substrat ist jedoch insofern besonders vorteilhaft, als ein stabiles bruchfestes Substratmaterial verwendet werden kann, das bei kleinem Querschnitt der Lichtleitfaserschicht 22 hohe Festigkeit verleiht. Aus der Halbleitertechnologie stehen bewährte Verfahren zur mikromechanischen Bearbeitung entsprechender Schichten zur Verfügung, so daß es durchaus möglich ist, das Schichtpaket 22,80 in den gewünschten extrem kleinen Abmessungen und in guter Festigkeit herzustellen.

Von den für die CVD-Diamantbeschichtung gebräuchlichen Materialien ist insbesondere Molybdän im Rahmen der vorliegenden Erfindung geeignet, wenn dessen Oberfläche so bearbeitet ist, daß sie spiegelnd glänzt. Das für andere Zwecke ebenfalls gebräuchliche Substratmaterial Silizium ist für eine Diamant-Lichtleitfaser hingegen ungeeignet, weil der Brechungsindex eines transparenten Substratmaterials niedriger als der des Lichtleitfasermaterials sein muß. Generell kommen für das Substrat Materialien in Betracht, die einen im Vergleich zu dem Material der Lichtleitfaser kleineren Brechungsindex und eine möglichst geringe Dämpfung aufweisen, wie beispielsweise Metall, Quarz, Saphir oder Keramik.

Die Figuren 11 und 12 zeigen im Querschnitt unterschiedliche Anordnungen der Lichtleitfaser bezüglich des Substrats 80. Bei der in Figur 11 dargestellten Anordnung ist das Substrat 80 beidseitig mit im Querschnitt rechteckigen Lichtleitfasern 22 versehen, wobei diese - ähnlich wie bei der Ausführungsform nach Figur 1 - eine einleitende Faserstrecke 20 und eine rückleitende Faserstrecke 21 bilden. Die Umlenkung am distalen Ende kann beispielsweise mittels eines prismatischen Reflektors ähnlich wie bei Figur 3 und 4 erfolgen.

Bei der in Figur 12 dargestellten Variante befinden sich auf einer Seite des Substrats sowohl die einleitende Faserstrecke 20 als auch die ausleitende Faserstrecke 21. In diesem Fall erfolgt die Umlenkung am distalen Ende zweckmäßigerweise durch eine U-förmige Formgebung der Lichtleitfaser 22 ähnlich wie bei Figur 5. Sowohl bei Figur 11 als auch bei Figur 12 ist auf der Innenseite der Kanüle 18 eine Membran wie bei den Figuren 5 und 6 vorgesehen. Der Vergleich der Figuren 11 und 12 zeigt, daß die Perforationslöcher 19 der Kanüle 18 ganz unterschiedliche Größen haben können. Auch ihre Form kann unterschiedlich sein. Neben einer runden Gestaltung kommt auch eine längliche schlitzartige Formgebung der Perforationslöcher 19 in Betracht.

Wie in Figur 11 zu erkennen ist, kann der gewünschte Abstand zwischen der Lichtleitfaser 22 und der Membran 42 bei dieser Ausführungsform durch eine geeignete Querschnittsformgebung des Substrats 80 sichergestellt werden.

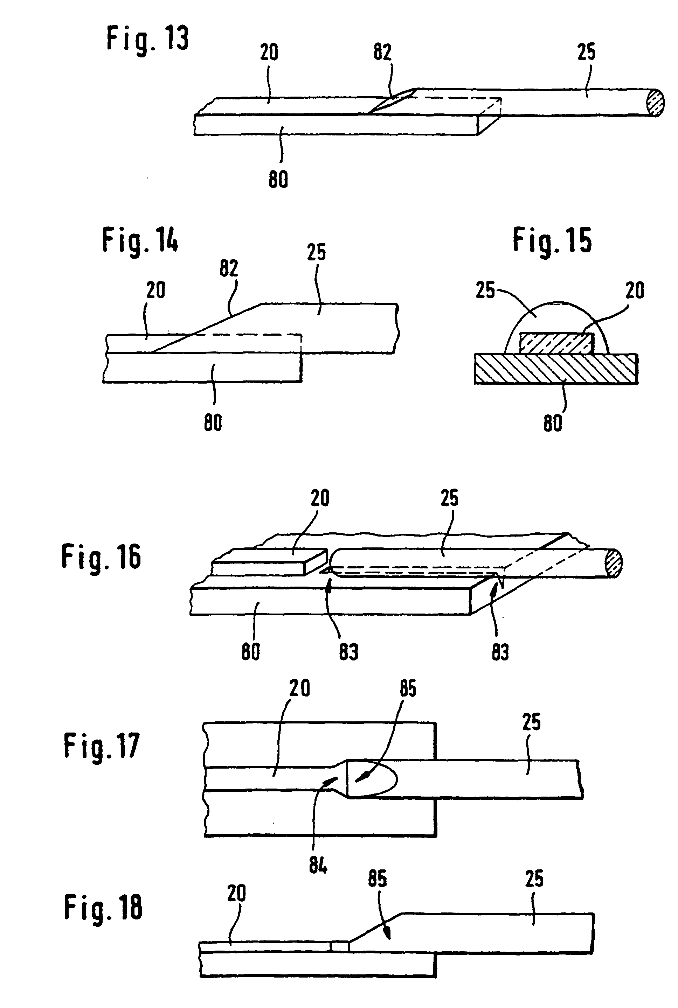

Die Verwendung eines auf einem Substrat befindlichen dünnen Schichtmaterials wirft besondere Probleme hinsichtlich der Ankopplung des von einer Elektronikeinheit (wie bei den Figuren 1 und 2) über ein faseroptisches Kabel zugeführten Lichts auf. In den Figuren 13 bis 18 sind verschiedene Ausführungsformen dieser Ankopplung dargestellt, wobei jeweils die einleitende Faserstrecke eines für die Zuführung des Lichtes dienenden faseroptischen Kabels (wie bei Figur 3) mit 25 und die anschließende einleitende Faserstrecke der Meßsonde mit 20 bezeichnet ist. Selbstverständlich können die gleichen Prinzipien auch für die Überkopplung des Lichts zwischen den entsprechenden rückleitenden Faserstrecken 26,21 bzw. eine Lichtleitfaser 22, die wie bei Figur 10 gleichzeitig zum Einleiten und Rückleiten des Lichts dient, verwendet werden.

Bei der in Figur 13 dargestellten Ausführungsform ist die Faserstrecke 25 des zur Zuführung verwendeten faseroptischen Kabels an die im Querschnitt rechteckige Faserstrecke 20 der Lichtleitfaser 22 dadurch angekoppelt, daß sie randseitig angepreßt ist. Dieses Verfahren eignet sich in Fällen, bei denen eine der miteinander zu verkoppelnden optischen Fasern (hier die Faserstrecke 25) im Vergleich zu der anderen Faser (hier 20) eine geringere Härte hat. Dies gilt insbesondere, wenn die eine Lichtleitfaser 20 aus Diamant und die andere Lichtleitfaser 25 aus einem vergleichsweise weichen Material wie insbesondere einem Silberhalogenid hergestellt ist. Besonders geeignet ist AgBrₓCl₁₋ₓ mit x=0,75. Die aufgepreßte Faser 25 weist endseitig eine Abschrägung 82 auf, durch die ein weitgehend kontinuierlicher Strahlungsübertritt zwischen den Fasern 25 und 20 erreicht wird. Die Ausführungsform erlaubt eine einfache justagefreie Realisierung der optischen Ankopplung.

Die in Figur 14 in Seitenansicht und in Figur 15 in Vorderansicht dargestellte Ausführungsform stimmt weitgehend mit der gemäß Figur 13 überein, wobei jedoch die optische Faser 25 auf die optische Faser 20 unter Kontaktierung des Substrats 80 aufgepreßt ist, so daß die Faser 25 die Faser 20 endseitig vollständig umschließt. Dies erleichtert die Herstellung in großen Stückzahlen.

Die in den Figuren 13 bis 15 dargestellte Preßkopplung zur Verbindung der Lichtleiter ist grundsätzlich auch in Fällen verwendbar, bei denen die in der Kanüle verlaufende Faserstrecke sich nicht auf einem Substrat befindet, sondern freitragend in der Kanüle 18 verläuft. Beispielsweise kann ein Lichtleiter in Form einer Diamantnadel in eine Silberhalogenidfaser eingepreßt werden.

Bei der in Figur 16 dargestellten Ausführungsform ist die optische Ankopplung ohne unmittelbaren Kontakt zwischen den Fasern 25 und 20 realisiert. Das dargestellte Ende der Faser 25 ist in eine in dem Substrat 80 vorgesehene Nut eingelegt, die als Positioniergraben 83 bezeichnet wird. Sie dient dazu, die Faser 25 exakt vor der gegenüberliegenden Stirnseite der Faser 20, die auf dem Substrat 80 fixiert ist, zu positionieren. Zwischen den gegenüberliegenden Stirnseiten der Fasern 20 und 25 ist ein Freiraum mit freier Strahlausbreitung vorgesehen, wobei das der Faser 20 zugewandte Ende der Faser 25 abgerundet ist, um einen fokussierenden Linseneffekt zu bewirken.

Die Figuren 17 und 18 zeigen in Aufsicht bzw. Seitenansicht eine Ausführungsform, bei der die in der Sonde verlaufende Faserstrecke 20 in Querrichtung aufgeweitete Endabschnitte 84 aufweist, an die stirnseitig unter Querschnittsanpassung die Faserstrecke 25 eines zur Lichtzuführung dienenden faseroptischen Kabels mit sich verjüngendem Taper-Übergang 85 angefügt ist. Durch diese Gestaltung wird ein möglichst effektiver Strahlungsübergang an der im Querschnitt sprungfrei ausgebildeten Übergangsstelle bewirkt.

## Patentansprüche

1. Analysevorrichtung zur Bestimmung eines Analyten in vivo im Körper eines Patienten, umfassend
eine Meßsonde mit einer in die Haut einstechbaren Kanüle (18) und einer in der Kanüle (18) verlaufenden Lichtleitfaser (22), durch die von einer Lichtquelle (8) ausgehendes, in die Lichtleitfaser (22) eingekoppeltes Licht in die Kanüle (18) und somit bei in die Haut eingestochener Meßsonde in das Körperinnere geleitet wird, wobei das in der Lichtleitfaser (22) transportierte Licht in der Meßsonde (3) durch unmittelbare reagenzfreie Wechselwirkung mit die Lichtleitfaser in dem Körperinneren umgebender Flüssigkeit eine für die Gegenwart des Analyten charakteristische Veränderung erfährt und
eine Meß- und Auswerteeinheit (4), um die Veränderung zu messen und aus der Veränderung eine Information über die Gegenwart des Analyten in dem Körper zu gewinnen,
wobei
die Wand der Kanüle (18) mindestens auf einem Teilabschnitt ihrer in die Haut einstechbaren Länge mit Löchern perforiert und dadurch für interstitielle Flüssigkeit penetrierbar ist, und
die Lichtleitfaser (22) einen Meßabschnitt (30) einschließt, der von einer Teillänge der Lichtleitfaser (22) innerhalb des penetrierbaren Teilabschnitts der Kanüle gebildet wird,
so daß interstitielle Flüssigkeit durch die Kanülenwand zu dem in der Kanüle (18) verlaufenden Meßabschnitt (30) der Lichtleitfaser gelangt.

2. Analysevorrichtung nach Anspruch 1, **dadurch gekennzeichnet, daß** der Querschnitt der Lichtleitfaser (22) in dem Meßabschnitt (30) variiert.

3. Analysevorrichtung nach Anspruch 1 oder 2, **dadurch gekennzeichnet, daß** in der Kanüle (18) in dem Meßabschnitt (30) nur eine Lichtleitfaserstrecke (45) verläuft, an deren distalem Ende eine Verspiegelung (47) vorgesehen ist, durch die das Licht in die gleiche Lichtleitfaserstrecke (45) zurückreflektiert wird.

4. Analysevorrichtung nach Anspruch 1 oder 2, **dadurch gekennzeichnete daß** in der Kanüle (18) in dem Meßabschnitt (30) zwei Lichtleitfaserstrecken (20,21) parallel verlaufen, wobei das Licht durch die eine Lichtleitfaserstrecke (20) in Richtung auf das distale Ende (23) der Kanüle (18) transportiert, in dem Bereich des distalen Endes (23) der Kanüle (18) umgelenkt und durch die andere Lichtleitfaserstrecke (21) ausgeleitet wird.

5. Analysevorrichtung nach Anspruch 4, **dadurch gekennzeichnet, daß** zur Umlenkung des Lichts im Bereich des distalen Endes (23) der Kanüle (18) ein prismatischer Reflektor (24) angeordnet ist.

6. Analysevorrichtung nach einem der vorhergehenden Ansprüche, **dadurch gekennzeichnet, daß** die Lichtleitfaser (22) wenigstens in dem Meßabschnitt von einer Beschichtung umgeben ist.

7. Analysevorrichtung nach Anspruch 6, **dadurch gekennzeichnet, daß** die Beschichtung (41) metallisch ist.

8. Analysevorrichtung nach Anspruch 6, **dadurch gekennzeichnet, daß** die Beschichtung (41) polymer ist.

9. Analysevorrichtung nach einem der Ansprüche 6 bis 8, **dadurch gekennzeichnet, daß** die Beschichtung (41) Analyt-anreichernde Eigenschaften hat.

10. Analysevorrichtung nach Anspruch 6, **dadurch gekennzeichnet, daß** die Beschichtung (60) aus synthetischem Diamant einschließlich diamantartigem Kohlenstoff (diamond like carbon, DLC) besteht.

11. Analysevorrichtung nach einem der Ansprüche 6 bis 10, **dadurch gekennzeichnet, daß** die Beschichtung (60) einen mindestens ebenso großen Brechungsindex wie der Lichtleiter hat.

12. Analysevorrichtung nach einem der vorhergehenden Ansprüche, **dadurch gekennzeichnet, daß** der Meßabschnitt (30) der Lichtleitfaser (22) von einer semipermeablen Membran (42) derartig umhüllt ist, daß die interstitielle Flüssigkeit in dem Meßabschnitt nur durch die Membran (42) an die Oberfläche der Lichtleitfaser (22) gelangt.

13. Analysevorrichtung nach Anspruch 12, **dadurch gekennzeichnet, daß** die Membran (42) innerhalb der Kanüle (18) angeordnet ist.

14. Analysevorrichtung nach Anspruch 12, **dadurch gekennzeichnet, daß** die Membran (42) von der äußeren Oberfläche der Lichtleitfaser im wesentlichen beabstandet ist.

15. Analysevorrichtung nach Anspruch 14, **dadurch gekennzeichnet, daß** die Beabstandung mittels einer Beschichtung der Lichtleitfaser (22), mittels eines in Längsrichtung in der Kanüle (18) zwischen der Lichtleitfaser (22) und der Membran (42) verlaufenden Metalldrahtes (61) oder mittels eines in seinen Querschnittsdimensionen über die Abmessungen der Lichtleitfaser (22) hinausragenden Substrates (80) bewirkt wird.

16. Analysevorrichtung nach einem der Ansprüche 12 bis 15, **dadurch gekennzeichnet, daß** die Membran (42) auf Polycarbonat basiert.

17. Analysevorrichtung nach einem der vorhergehenden Ansprüche, **dadurch gekennzeichnet, daß** die Wechselwirkung des in der Lichtleitfaser (22) transportierten Lichts mit der interstitiellen Flüssigkeit auf dem Eindringen eines evaneszenten Feldes in die interstitielle Flüssigkeit basiert.

18. Analysevorrichtung nach einem der vorhergehenden Ansprüche, **dadurch gekennzeichnet, daß** die Wellenlänge des von der Lichtquelle (8) ausgehenden Lichts zwischen 7 *µ*m und 13 *µ*m liegt.

19. Analysevorrichtung nach einem der vorhergehenden Ansprüche, **dadurch gekennzeichnet, daß** die Lichtquelle (8) mindestens einen Quantenkaskadenlaser (31-33) aufweist.

20. Analysevorrichtung nach einem der Ansprüche 1 bis 9 und 11 bis 19, **dadurch gekennzeichnet, daß** das Material der Lichtleitfaser (22) ausgewählt ist aus der Gruppe bestehend aus Silberhalogenid, Chalcogenid-Glas, synthetischem Diamant einschließlich diamantartigem Kohlenstoff (diamond like carbon, DLC), Silizium und Germanium.

21. Analysevorrichtung nach einem der vorhergehenden Ansprüche, **dadurch gekennzeichnet, daß** die Lichtleitfaser (22) aus einem durch Abscheiden aus der Gasphase auf einem Substrat erzeugten Schicht herausgearbeitet ist.

22. Analysevorrichtung nach Anspruch 21, **dadurch gekennzeichnet, daß** die Lichtleitfaser zusammen mit dem Substrat (80) in die Kanüle (18) integriert ist.

23. Analysevorrichtung nach einem der vorhergehenden Ansprüche, **dadurch gekennzeichnet, daß** an der Lichtleitfaser (22) im Bereich des proximalen Endes der Kanüle (18) ein Heizelement (68) und/oder ein Temperaturmeßelement (71) angebracht ist.

24. Analysevorrichtung nach einem der vorhergehenden Ansprüche, **dadurch gekennzeichnet, daß** an die Lichtleitfaser (22,20) eine optische Faser (25) mit im Vergleich zu dem Material der Lichtleitfaser (22,20) geringerer Härte durch Pressen angefügt ist.

25. Analysevorrichtung nach einem der vorhergehenden Ansprüche, **dadurch gekennzeichnet, daß** die Löcher der Perforation der Kanüle ein Durchmesser zwischen etwa 1 µm und etwa 100 µm haben.

## Claims

1. Analysis device for determining an analyte in vivo in the body of a patient comprising
a measuring probe having a hollow needle (18) adapted to be pierced into the skin and with an optical fiber (22) which runs inside the hollow needle (18) and by which light coupled from a light source (8) into the optical fiber (22) is passed into the hollow needle (18) and thus into the body when the measuring probe is pierced into the skin, wherein the light transported through the optical fiber (22) is modified in the measuring probe (3) by direct reagent free interaction with the liquid surrounding the optical fiber within the body, said modification being characteristic of the presence of the analyte and
a measuring and evaluation unit (4) to measure the modification and to derive, on the basis of the modification, information concerning the presence of the analyte in the body,
wherein _
the wall of the hollow needle (18) is, at least along a partial section of its length which is adapted for penetration into the skin, perforated with holes through which the interstitial liquid can penetrate,
the optical fiber (22) comprises a measuring section (30) formed by a partial length of the optical fiber (22) within the penetrable partial section of the hollow needle,
so that interstitial liquid reaches through the wall of the hollow needle the measuring section (30) of the optical fiber, which section extends in the hollow needle (18).

2. Analysis device according to claim 1, **characterized in that** the cross-section of the optical fiber (22) varies along the measuring section (30).

3. Analysis device according to claim 1 or 2, **characterized in that** only one optical fiber track (45) runs inside the hollow needle (18) in the measuring section (30), the distal end of the optical fiber track (45) being provided with a mirror (47) which reflects the light back along the same optical fiber track (45).

4. Analysis device according to claim 1 or 2, **characterized in that** two parallel optical fiber tracks (20, 21) extend in the hollow needle (18) in the measuring section (30), wherein the light is transported through the one optical fiber track (20) towards the distal end (23) of the hollow needle (18) and is deflected at the distal end (23) of the hollow needle (18) to return through the other optical fiber track (21) .

5. Analysis device according to claim 4, **characterized in that** a prismatic reflector (24) is arranged at the distal end (23) of the hollow needle (18) to deflect the light.

6. Analysis device according to any one of the preceding claims, **characterized in that** the optical fiber (22) is surrounded, at least in the measuring section, by a coating.

7. Analysis device according to claim 6, **characterized in that** the coating (41) is metallic.

8. Analysis device according to claim 6, **characterized in that** the coating (41) is a polymer.

9. Analysis device according to any one of the claims 6 through 8, **characterized in that** the coating (41) has analyte enriching properties.

10. Analysis device according to claim 6, **characterized in that** the coating (60) consists of synthetic diamond including diamond like carbon (DLC).

11. Analysis device according to any one of the claims 6 through 10, **characterized in that** the coating (60) has an index of refraction which is at least as large as that of the light guide.

12. Analysis device according to any one of the preceding claims, **characterized in that** the measuring section (30) of the optical fiber (22) is jacketed by a semipermeable membrane (42) such that the interstitial liquid in the measuring section only gains access to the surface of the optical fiber (22) through the membrane (42).

13. Analysis device according to claim 12, **characterized in that** the membrane (42) is arranged within the hollow needle (18).

14. Analysis device according to claim 12, **characterized in that** the membrane (42) is essentially separated from the outer surface of the optical fiber.

15. Analysis device according to claim 14, **characterized in that** the separation is effected by a coating on the optical fiber (22), or by a metallic wire (61) travelling in the longitudinal direction within the hollow needle (18) between the optical fiber (22) and the membrane (42), or by a substrate (80) having a cross-sectional dimension projecting past the dimension of the optical fiber (22).

16. Analysis device according to any one of claims 12 through 15, **characterized in that** the membrane (42) is based on polycarbonate.

17. Analysis device according to any one of the preceding claims, **characterized in that** the interaction of the light transported in the optical fiber (22) with the interstitial liquid is due to the penetration of an evanescent field into the interstitial liquid.

18. Analysis device according to any one of the preceding claims, **characterized in that** the wavelength of the light from the light source (8) is between 7 µm and 13 µm.

19. Analysis device according to any one of the preceding claims, **characterized in that** the light source (8) comprises at least one quantum cascade laser (31 through 33).

20. Analysis device according to any one of claims 1 through 9 and 11 through 19, **characterized in that** the material of the optical fiber (22) is selected from the group consisting of silver halide, chalcogenic glass, synthetic diamond, diamond-like carbon, silicon, and germanium.

21. Analysis device according to any one of the preceding claims, **characterized in that** the optical fiber (22) is fashioned from a layer produced by deposition from the gas phase onto a substrate.

22. Analysis device according to claim 21, **characterized in that** the optical fiber is integrated together with the substrate (80) into the hollow needle (18).

23. Analysis device according to any one of the preceding claims, **characterized in that** a heating element (68) and/or a temperature measuring element (71) is disposed on the optical fiber (22) near the proximal end of the hollow needle (18).

24. Analysis device according to any one of the preceding claims, **characterized in that** a light guide fiber (25), having a hardness which is less than that of the material from which the optical fiber (22,20) is made, is joined to the optical fiber (22,20) by pressing.

25. Analysis device according to any of the preceding claims, **characterized in that** the holes of the perforated section of the hollow needle have a cross-section between about 1 µm and 100 µm.

## Revendications

1. Dispositif d'analyse pour la détermination d'un analyte in vivo dans le corps d'un patient, comprenant
une sonde de mesure équipée d'une canule (18) qui peut être enfoncée dans la peau et une fibre de guidage de lumière (22) s'étendant dans la canule (18), par laquelle la lumière alimentée dans la fibre de guidage de lumière (22) à partir d'une source de lumière (8) est guidée dans la canule (18) et par conséquent à l'intérieur du corps via la sonde de mesure enfoncée dans la peau, la lumière transportée dans la fibre de guidage de lumière (22) subissant, dans la sonde de mesure (3), via une interaction directe exempte de réactif avec le liquide entourant la fibre de guidage de lumière à l'intérieur du corps, une modification caractéristique pour la présence de l'analyte et
une unité de mesure et d'évaluation (4) pour mesurer la modification et pour obtenir des informations, à partir de la modification, quant à la présence de l'analyte dans le corps,
dans lequel
la paroi de la canule (18) est perforée de trous, au moins sur une section partielle de sa longueur apte à s'enfoncer dans la peau, si bien que du liquide interstitiel est à même de pénétrer à travers ladite paroi, et
la fibre de guidage de lumière (22) renferme une section de mesure (30) qui est formée par une longueur partielle de la fibre de guidage de lumière (22) dans les limites de la section partielle de la canule manifestant une aptitude à la pénétration,
si bien que du liquide interstitiel aboutit, en traversant la paroi de la canule, à la section de mesure (30) de la fibre de guidage de lumière s'étendant dans la canule (18).

2. Dispositif d'analyse selon la revendication 1, **caractérisé en ce que** la section transversale de la fibre de guidage de lumière (22) varie dans la section de mesure (30).

3. Dispositif d'analyse selon la revendication 1 ou 2, **caractérisé en ce que**, dans la canule (18), un seul tronçon de fibre de guidage de lumière (45) s'étend dans la section de mesure (30), à l'extrémité distale duquel on prévoit un dépôt de couches réfléchissantes (47), par lequel la lumière est réfléchie en retour dans le même tronçon de fibre de guidage de lumière (45).

4. Dispositif d'analyse selon la revendication 1 ou 2, **caractérisé en ce que**, dans la canule (18), deux tronçons de fibres de guidage de lumière (20, 21) s'étendent parallèlement dans la section de mesure (30), la lumière étant transportée par le premier tronçon de fibre de guidage de lumière (20) dans la direction de l'extrémité distale (23) de la canule (18), étant déviée dans la zone de l'extrémité distale (23) de la canule (18) et étant dirigée vers l'extérieur via l'autre tronçon de fibre de guidage de lumière (21).

5. Dispositif d'analyse selon la revendication 4, **caractérisé en ce que**, pour la déviation de la lumière dans la zone de l'extrémité distale (23) de la canule (18), on dispose un réflecteur en forme de prisme (24).

6. Dispositif d'analyse selon l'une quelconque des revendications précédentes, **caractérisé en ce que** la fibre de guidage de lumière (22) est entourée, au moins dans la section de mesure, d'un revêtement.

7. Dispositif d'analyse selon la revendication 6, **caractérisé en ce que** le revêtement (41) est métallique.

8. Dispositif d'analyse selon la revendication 6, **caractérisé en ce que** le revêtement (41) est polymère.

9. Dispositif d'analyse selon l'une quelconque des revendications 6 à 8, **caractérisé en ce que** le revêtement (41) possède des propriétés enrichissant l'analyte.

10. Dispositif d'analyse selon la revendication 6, **caractérisé en ce que** le revêtement (60) est constitué d'un diamant synthétique, y compris du carbone diamantin (diamond like carbon, DLC).

11. Dispositif d'analyse selon l'une quelconque des revendications 6 à 10, **caractérisé en ce que** le revêtement (60) possède un indice de réfraction au moins aussi élevé que celui de la fibre de guidage de lumière.

12. Dispositif d'analyse selon l'une quelconque des revendications précédentes, **caractérisé en ce que** la section de mesure (30) de la fibre de guidage de lumière (22) est entourée d'une membrane semi-perméable (42) de telle sorte que le liquide interstitiel, dans la section de mesure, ne parvient à la surface de la fibre de guidage de lumière (22) qu'en traversant la membrane (42).

13. Dispositif d'analyse selon la revendication 12, **caractérisé en ce que**, la membrane (42) est disposée à l'intérieur de la canule (18).

14. Dispositif d'analyse selon la revendication 12, **caractérisé en ce que** la membrane (42) est disposée essentiellement à l'écart de la surface externe de la fibre de guidage de lumière.

15. Dispositif d'analyse selon la revendication 14, **caractérisé en ce qu'**on obtient l'écartement à l'aide d'un revêtement de la fibre de guidage de lumière (22), à l'aide d'un fil métallique (61) s'étendant en direction longitudinale dans la canule (18) entre la fibre de guidage de lumière (22) et la membrane (42) ou via un substrat (80) faisant saillie, quant à ses dimensions en section transversale, par rapport aux dimensions de la fibre de guidage de lumière (22).

16. Dispositif d'analyse selon l'une quelconque des revendications 12 à 15, **caractérisé en ce que** la membrane (42) est à base de polycarbonate.

17. Dispositif d'analyse selon l'une quelconque des revendications précédentes, **caractérisé en ce que** l'interaction de la lumière transportée dans la fibre de guidage de lumière (22) avec le liquide interstitiel se base sur l'apparition d'un champ évanescent dans le liquide interstitiel.

18. Dispositif d'analyse selon l'une quelconque des revendications précédentes, **caractérisé en ce que** la longueur d'onde de la lumière émise par la source de lumière (8) se situe entre 7 µm et 13 µm.

19. Dispositif d'analyse selon l'une quelconque des revendications précédentes, **caractérisé en ce que** la source de lumière (8) présente au moins un laser quantique en cascade (31 - 33).

20. Dispositif d'analyse selon l'une quelconque des revendications 1 à 9 et 11 à 19, **caractérisé en ce que** la matière de la fibre de guidage de lumière (22) est choisie parmi le groupe constitué par un halogénure d'argent, du verre à base de chalcogénure, du diamant synthétique, y compris du carbone diamantin (diamond like carbon, DLC), du silicium et du germanium.

21. Dispositif d'analyse selon l'une quelconque des revendications précédentes, **caractérisé en ce qu'**on obtient la fibre de guidage de lumière (22) à partir d'un traitement d'une couche générée par précipitation en phase gazeuse sur un substrat.

22. Dispositif d'analyse selon la revendication 21, **caractérisé en ce que** la fibre de guidage de lumière est intégrée avec le substrat (80) dans la canule (18).

23. Dispositif d'analyse selon l'une quelconque des revendications précédentes, **caractérisé en ce qu'**un élément de chauffage (68) et/ou un élément de mesure de la température (71) sont appliqués sur la fibre de guidage de lumière (22) dans la zone de l'extrémité proximale de la canule (18).

24. Dispositif d'analyse selon l'une quelconque des revendications précédentes, **caractérisé en ce qu'**une fibre optique (25), qui possède une dureté inférieure à celle de la matière de la fibre de guidage de lumière (22, 20), est jointe par pression à la fibre de guidage de lumière (22, 20).

25. Dispositif d'analyse selon l'une quelconque des revendications précédentes, **caractérisé en ce que** les trous de la perforation de la canule possèdent un diamètre entre environ 1 µm et environ 100 µm.
